# EUROPÄISCHE PATENTSCHRIFT

(11) **EP 2 732 754 B1**
(45) Veröffentlichungstag und Bekanntmachung des Hinweises auf die Patenterteilung: **21.10.2015**
(21) Anmeldenummer: 13005333.3
(22) Anmeldetag: 13.11.2013
(51) Int. Cl.: A61B 1/005, A61B 1/00

(54) **Flexibler Endoskopschaft sowie derartiges Endoskop**
Flexible endoscope shaft and endoscope of this sort
Tige d'endoscope flexible et endoscope de ce type

(30) Priorität: 15.11.2012 DE 102012022442
(43) Veröffentlichungstag der Anmeldung: 21.05.2014
(73) Patentinhaber: Karl Storz GmbH & Co. KG, 78532 Tuttlingen (DE)
(72) Erfinder: Wimmer, Viktor, 83370 Seeon (DE); Ayrenschmalz, Robert, 84100 Niederaichbach (DE)
(74) Vertreter: Hofmeister, Frank

(56) Entgegenhaltungen:
- EP-A1- 2 524 645
- DE-A1- 4 102 211
- DE-A1- 4 317 914
- US-A1- 2008 300 462
- US-A1- 2009 118 584

## Beschreibung

Die Erfindung betrifft einen flexiblen Endoskopschaft mit einem Schaftkörper, der in Längsrichtung des Endoskopschaftes betrachtet aus mindestens zwei Schaftabschnitten mit unterschiedlichem Flexibilitätsgrad besteht, wobei mindestens ein Schaftabschnitt aus mindestens einem Federelement besteht und wobei alle Federelemente der Schaftabschnitte, die in Längsrichtung des Schaftkörpers betrachtet proximalseitig vom äußeren distalen Schaftabschnitt angeordnet sind, als in Längsrichtung des Schaftkörpers verlaufende, reversibel verformbare Federstäbe ausgebildet sind. Weiterhin betrifft die Erfindung ein Endoskop, das einen flexiblen Endoskopschaft der voranstehend genannten Art aufweist.

Flexible Endoskope, das heißt Endoskope mit einem flexiblen Endoskopschaft werden in der Medizin und zu technischen Zwecken eingesetzt, um innenliegende verzweigte oder verschlungene Körperpartien oder Maschinenteile zu untersuchen.

Von starren Endoskopen unterscheiden sich flexible Endoskope dadurch, dass der Endoskopschaft bei einem flexiblen Endoskop eine gebogene, gekrümmte oder sogar schlaufenförmige Form annehmen kann. Aufgrund dieser Flexibilität des Endoskopschaftes eignen sich flexible Endoskope besonders gut für medizinische Operations- und Untersuchungszwecke von inneren Körperpartien, die verzweigte oder verschlungene Strukturen aufweisen, wie beispielsweise der Magen- und Darmbereich oder die Atemwege.

Aus der Praxis sind verschiedene Ausführungsformen zur Ausbildung flexibler Endoskopschäfte bekannt.

Aus der DE 199 08 152 A1 ist ein Endoskop bekannt, in dessen Endoskopschaft in Längsrichtung verlaufende biegsame Stäbe angeordnet sind. Diese biegsamen Stäbe ermöglichen es dem Operateur, den Endoskopschaft vor der Operation durch manuelles Verbiegen in einen gewünschten Krümmungsverlauf zu überführen, so dass der Endoskopschaft durch eine Körperöffnung bis zum Untersuchungsgebiet vorgeschoben werden kann. Aufgrund der Steifigkeit der Stäbe behält der Endoskopschaft diesen vorab aufgegebenen Krümmungsverlauf während des Einsatzes dauerhaft bei.

Diese Steifigkeit der die Formgebung des Endoskopschaftes ermöglichenden Biegestäbe gewährleistet zwar eine für die Verwendung wichtige innere Steifigkeit des Endoskopschaftes, jedoch schränkt sie auch die Einsatzmöglichkeiten eines derartig ausgebildeten flexiblen Endoskops ein, da der vorgegebene Krümmungsverlauf des Endoskopschaftes während der Operation nicht veränderbar ist, um beispielsweise seitliche Abzweigungen oder dergleichen zu untersuchen

Dokument US 2009/118584 A1 offenbart ebenfalls ein Endoskop, in dessen flexiblen Endoskopschaft mehrere, in Längsrichtung verlaufende biegbare Stäbe angeordnet sind

Ein gattungsgemäßes flexibles Endoskop ist aus der EP 1 658 805 B1 bekannt. Bei diesem bekannten flexiblen Endoskop besteht der Endoskopschaft aus mehreren Schaftabschnitten, die jeweils aus einer Schraubenfeder gebildet sind. Durch die Wahl unterschiedlicher Schraubenfedern lässt sich der Flexibilitätsgrad der einzelnen Schaftabschnitte individuell einstellen. Die reversibel verformbaren Schraubenfedern dieses bekannten Endoskops ermöglichen einen flexiblen Endoskopschaft, der auch während der Untersuchung in jede gewünschte Richtung verstellen lässt.

Die Ausbildung der Federelemente der einzelnen Schaftabschnitte als Schraubenfedern hat den Nachteil, dass die Reinigung der engen Schraubenfedergänge ziemlich aufwendig ist. Darüber hinaus ist die Fertigung der unterschiedlichen Schraubenfedern sehr aufwendig und kostenintensiv.

Davon ausgehend liegt der Erfindung die **Aufgabe** zugrunde, einen flexiblen Endoskopschaft der eingangs genannten Art zu schaffen, der einfach und kostengünstig herstellbar ist.

Die **Lösung** dieser Aufgabenstellung erfolgt erfindungsgemäß über die Merkmale des kennzeichnenden Teils des Anspruchs 1.

Durch die Ausbildung der Federelemente als Federstäbe aus flachem oder rundem Federdraht lässt sich der Schaftkörper einfach und kostengünstig herstellen.

Um die Formstabilität und Torsionssteifigkeit der einzelnen Schaftabschnitte hin zum proximalen Ende des Schaftkörpers zu steigern, weist in Längsrichtung des Schaftkörpers betrachtet von distal nach proximal jeder Schaftabschnitt mindestens einen zusätzlichen Federstab auf, der stoffschlüssig, insbesondere durch Laserschweißen, mit den anderen Federstäben verbunden ist, wobei der mindestens eine zusätzliche Federstab in Längsrichtung des Schaftkörpers verlaufend seitlich auf den anderen Federstäben des jeweiligen Schaftabschnitts angeordnet ist.

Weiterhin der Erfindung wird vorgeschlagen, dass jeder Schaftabschnitt der in Längsrichtung des Schaftkörpers betrachtet proximalseitig vom äußeren distalen Schaftabschnitt angeordneten Schaftabschnitte mindestens zwei Federstäbe aufweist, deren Längserstreckung jeweils bis zum proximalen Ende des Schaftkörpers reicht. Die Verwendung von mindestens zwei Federstäben für den nicht distalen Schaftabschnitt ermöglicht die Ausbildung eines Schaftkörpers, der eine ausreichende Biegesteifigkeit und Schubstabilität aufweist.

Weiterhin wird mit der Erfindung vorgeschlagen, dass die Federstäbe eines jeden Schaftabschnitts wendelförmig umeinander und/oder wendelförmig um die Federstäbe der in Längsrichtung des Schaftkörpers betrachtet distal gelegenen Schaftabschnitte gewickelt sind. Dieses wendelförmige Umeinanderwickeln der Federstäbe erhöhte die Formstabilität, Biegesteifigkeit und Torsionsstabilität der Schaftabschnitte. Um die Federstäbe in ihrer umeinandergewickelten Lage zueinander zu fixieren und ein Aufdrehen der Wicklung zu verhindern, wird weiterhin vorgeschlagen, dass die wendelförmig umeinander gewickelten Federstäbe abschnittweise, vorzugsweise punktuell, stoffschlüssig, vorzugsweise durch Laserschweißen, miteinander verbunden sind, wobei sich die Eigenschaften der einzelnen Schaftabschnitte dadurch variieren lassen, dass die umeinander gewickelten Federstäbe nur an einigen wenigen, oder aber an vielen Stellen miteinander verschweißt sind.

Das Umeinanderwickeln der Federstäbe hat weiterhin den Vorteil, dass es beim Biegen des Endoskopschaftes keine bevorzugte Biegerichtung gibt, sondern der Endoskopschaft mit etwa gleicher Kraft in alle Richtungen gebogen werden kann.

Die Eigenschaften der einzelnen Schaftabschnitte, beispielsweise hinsichtlich des Flexibilitätsgrades und der Biegesteifigkeit, lassen sich erfindungsgemäß unter anderem dadurch variieren, dass die Federstäbe der einzelnen Schaftabschnitte mit gleicher oder unterschiedlicher Windungszahl um die Federstäbe der in Längsrichtung des Schaftkörpers betrachtet distal oder proximal gelegenen Schaftabschnitte gewickelt sind. Mehr Windungen pro Längeneinheit bewirken dabei eine Versteifung des Schaftabschnittes, wohingegen weniger Windungen pro Längeneinheit den jeweiligen Schaftabschnitt flexibler ausbilden.

Gemäß einer bevorzugten Ausführungsform der Erfindung wird vorgeschlagen, dass der Flexibilitätsgrad der Federelemente der einzelnen Schaftabschnitte von distal nach proximal abnimmt, das heißt, dass der äußere distale Schaftabschnitt am flexibelsten ausgebildet ist und die Schaftabschnitte hin zum proximalen Ende des Schaftkörpers immer steifer werden.

Gemäß einer weiteren erfindungsgemäßen Ausführungsform wird vorgeschlagen, dass der äußerste distale Schaftabschnitt aus einzelnen gelenkig miteinander verbundenen Segmenten besteht.

Zum Verschwenken des äußeren distalen Schaftabschnitts wird mit der Erfindung vorgeschlagen, dass der äußerste distale Schaftabschnitt über eine am distalen Schaftabschnitt angreifende Zugmechanik, vorzugsweise mindestens einen Bowdenzug, verschwenkbar ist.

Zur Ausbildung des fertigen Endoskopschaftes wird mit der Erfindung vorgeschlagen, dass alle Schaftabschnitte mit einem elastischen Außenmantel, vorzugsweise einem Schlauch, ummantelbar sind.

Weiterhin wird mit der Erfindung vorgeschlagen, dass in dem Außenmantel in Längsrichtung des Schaftkörpers verlaufende Arbeitskanäle, beispielsweise zur Aufnahme optischer Komponenten, zusätzlicher medizinischer Instrumente, elektrischer Leitungen sowie der Zugmechanik, angeordnet sind.

Die Erfindung betrifft weiterhin ein flexibles Endoskop, das einen wie voranstehend beschrieben aufgebauten flexiblen Endoskopschaft aufweist.

Weitere Merkmale und Vorteile der Erfindung ergeben sich anhand der zugehörigen Zeichnungen, in denen mehrere Ausführungsbeispiele eines erfindungsgemäßen flexiblen Endoskopschafts nur beispielhaft dargestellt sind, ohne die Erfindung auf diese Ausführungsbeispiele zu beschränken. In den Zeichnungen zeigt:
- Fig. 1: eine Gesamtdarstellung eines Endoskops mit einem flexiblen Schaft;
- Fig. 2: eine vergrößerte Darstellung des Details II gemäß Fig. 1 in verschiedenen Arbeitsstellungen;
- Fig. 3: eine vergrößerte ausschnittweise schematische Detailansicht des distalen und mittleren Schaftabschnitts gemäß eines erfindungsgemäßen flexiblen Endoskopschafts;
- Fig. 4: eine vergrößerte ausschnittweise schematische Detailansicht des mittleren und proximalen Schaftabschnitts eines erfindungsgemäßen flexiblen Endoskopschafts und
- Fig. 5: eine ausschnittweise Seitenansicht eines erfindungsgemäßen flexiblen Endoskopschafts mit einem teilweise aufgeschobenen Außenmantel.

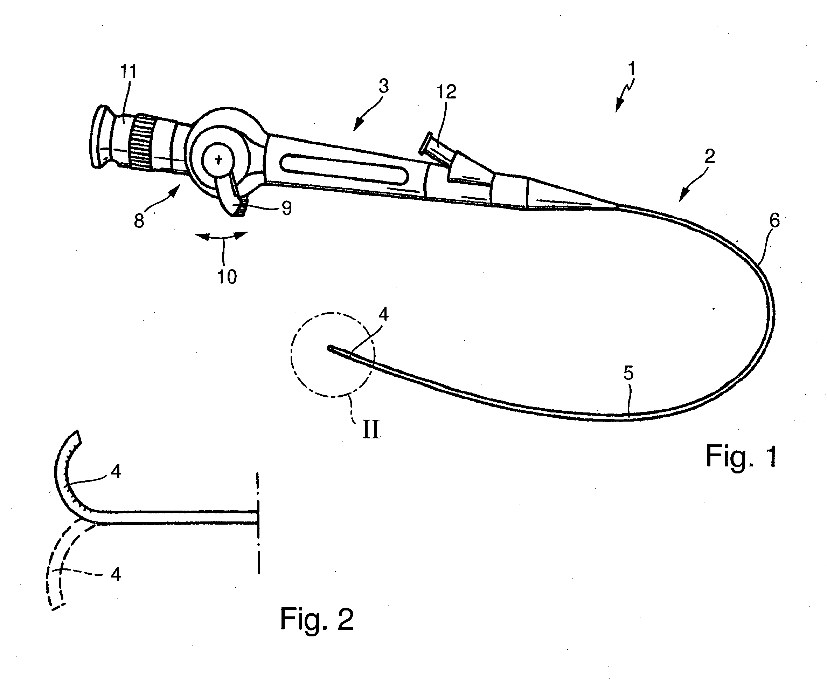

Die Abbildung Fig. 1 zeigt ein flexibles Endoskop 1 mit einem langen flexiblen Endoskopschaft 2, an dessen proximalem Ende eine Handhabe 3 angeordnet ist.

Der flexible Schaft 2 weist einen distalen Schaftabschnitt 4, einen mittleren Schaftabschnitt 5 sowie einen proximalen Schaftabschnitt 6 auf. Im Endoskopschaft 2 sind nicht dargestellte Arbeitskanäle angeordnet, die beispielsweise zur Aufnahme von Lichtleitfasern der Endoskopoptik, von elektrischen Leitungen und/oder zur Aufnahme von medizinischen Instrumenten, dienen.

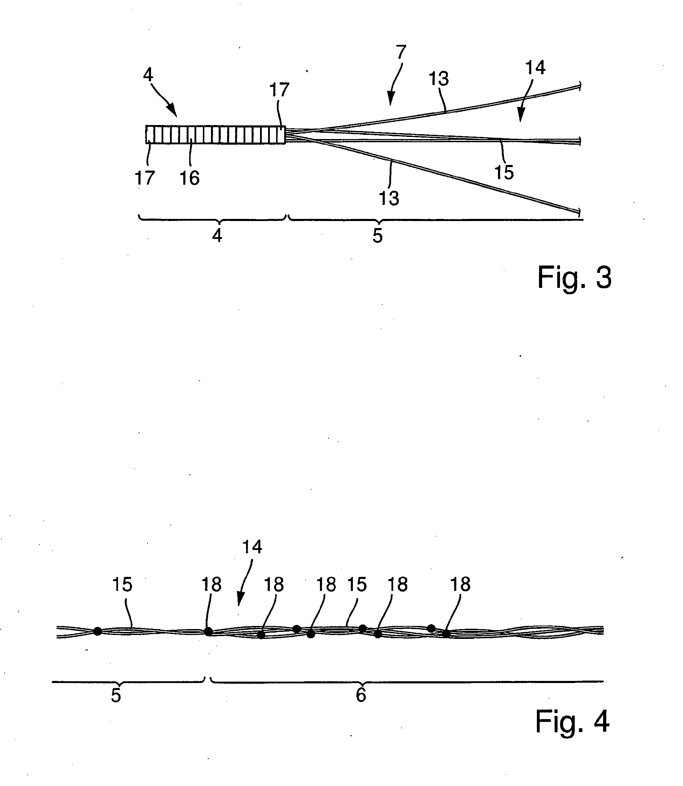

Der äußerste distale Schaftabschnitt 4, ist wie aus Fig. 2 ersichtlich, besonders flexibel ausgestaltet in verschiedene Richtungen verschwenkbar. Das Verschwenken des distalen Schaftabschnitts 4 erfolgt über eine in Fig. 3 und 4 dargestellte Zugmechanik 7 und eine an der Handhabe 3 angeordnete Steuereinrichtung 8, die beim in Fig. 1 dargestellten Ausführungsbeispiel als Stellhebel 9 ausgebildet ist, der in Richtung der Pfeile 10 verstellbar ist.

Die Handhabe 3 weist weiterhin am proximalen Ende eine Okular 11 sowie am distalen Ende der Handhabe 3 einen Arbeitskanaleingang 12 auf, über den beispielsweise ein medizinisches Instrument in einen Arbeitskanal des Endoskopschafts 2 einführbar ist.

Wie aus Fig. 2 ersichtlich, ist der distale Schaftabschnitt 4 des Endoskopschafts 2 aus der in Fig. 1 dargestellten Geradeausstellung nach oben und unten um nahezu 180° auslenkbar. Auch weitere Auslenkungen bis 210° sowie Auslenkungen des distalen Schaftabschnitts 4 in mehr als nur zwei Richtungen sind möglich.

Das Auslenken des distalen Schaftabschnitts 4 erfolgt über eine am distalen Schaftabschnitt 4 angreifende Zugmechanik 7, die bei der in Fig. 3 dargestellten Ausführungsform aus zwei am distalen Schaftabschnitt 4 festgelegten Bowdenzügen 13 besteht, die proximalseitig an der Steuereinrichtung 8 der Handhabe 3 festgelegt sind. Durch Betätigen des Stellhebels 9 der Steuereinrichtung 8 in Richtung der Pfeile 10 wird der distale Schaftabschnitt 4 in die gewünschte Richtung nach oben oder nach unten verschwenkt.

Zur Auslenkung des distalen Schaftabschnitts 4 in mehr als zwei Richtungen weist bei einer alternativen Ausführungsform des Endoskops 1 die Steuereinrichtung 8 zwei Stellhebel 9 auf, einen Stellhebel 9 zum Verschwenken des distalen Schaftabschnitts 4 nach oben und unten sowie einen Stellhebel 9 zum Verschwenken des distalen Schaftabschnitts 4 nach rechts und links. Mit diesen zwei Stellhebeln 9 sind dann auch alle Richtungen durch Kombination dieser Verschwenkrichtungen möglich.

Der Aufbau der Schaftabschnitte 4, 5 und 6 wird nachfolgend anhand der Abbildungen Fig. 3 bis 5 näher beschrieben.

Um dem Endoskopschaft 2 die erforderliche Flexibilität und gleichzeitig ausreichende Biegefestigkeit und Schubstabilität zu verleihen, weisen alle Schaftabschnitte 5, 6, die in Längsrichtung des Schaftkörpers betrachtet proximalseitig vom äußeren distalen Schaftabschnitt 4 angeordnet sind, reversibel verformbare Federelemente 14 auf.

Bei den in den Abbildungen Fig. 3 bis 6 dargestellten Ausführungsformen sind alle Federelemente 14 der Schaftabschnitte 5, 6, die in Längsrichtung des Schaftkörpers betrachtet proximalseitig vom äußeren distalen Schaftabschnitt 4 angeordnet sind, als in Längsrichtung des Schaftkörpers verlaufende, reversibel verformbare Federstäbe 15 ausgebildet.

Der äußerste distale Schaftabschnitt 4 besteht bei allen dargestellten Ausführungsformen aus einzelnen gelenkig miteinander verbundenen Segmenten 16.

Bei der in Fig. 3 dargestellten Ausführungsform ist der distale Schaftabschnitt 4 zum Anschluss an die Zugmechanik 7 distalseitig und proximalseitig zwischen zwei Bowdenzugaufnahmen 17 gelagert, wobei die proximalseitige Bowdenzugaufnahme 17 als Widerlager für den Bowdenzugmantel dient, während an der distalseitigen Bowdenzugaufnahme 17 das Bowdenzugseil gelagert ist. Durch Betätigen der Steuereinrichtung 8 an der Handhabe 3 lässt sich so der distale Schaftabschnitt 4 nach oben und unten verschwenken.

Der mittlere Schaftabschnitt 5 dieser dargestellten Ausführungsform besteht aus zwei Federstäben 15, die stoffschlüssig mit der proximalseitigen Bowdenzugaufnahme 17 des distalen Schaftabschnitts 4 verbunden sind. Wie weiterhin aus Fig. 4 ersichtlich, sind die beiden Federstäbe 15 des mittleren Schaftabschnitts 5 wendelförmig umeinander gewickelt und im weiteren Verlauf in Längsrichtung des mittleren Schaftabschnitts 5 abschnittweise, vorzugsweise punktuell, durch Schweißpunkte 18 miteinander verbunden und gegeneinander fixiert. Die stoffschlüssige Verbindung einzelner Federstäbe 15 miteinander erfolgt vorzugsweise mittels Laserschweißen.

Durch das wendelförmige Umeinanderwickeln der Federstäbe 15 wird die Biegefestigkeit und Torsionsstabilität der so ausgestalteten Schaftabschnitte 5, 6 erhöht. Das Umeinanderwickeln der Federstäbe 15 hat weiterhin den Vorteil, dass es beim Biegen des Endoskopschaftes 2 keine bevorzugte Biegerichtung gibt, sondern der Endoskopschaft 2 mit etwa gleicher Kraft in alle Richtungen gebogen werden kann.

Die Abbildung Fig. 4 zeigt eine vergrößerte ausschnittweise schematische Detailansicht des mittleren Schaftabschnitts 5 und des proximalen Schaftabschnitts 6, wie diese für die zuvor anhand der Abbildung Fig. 3 beschriebene Ausgestaltungsform des distalen Schaftabschnitts 4 verwendbar ist.

Im Übergang zum proximalen Schaftabschnitt 6 ist ein dritter, zusätzlicher Federstab 15 an die beiden Federstäbe 15 des mittleren Schaftabschnitts 5 stoffschlüssig, vorzugsweise mittels Laserschweißen, angeheftet. Wie weiterhin aus Fig. 4 ersichtlich, ist der zusätzliche Federstab 15 des proximalen Schaftabschnitts 6 in Längsrichtung des Schaftkörpers verlaufend seitlich auf den vom vorherigen mittleren Schaftabschnitt 5 kommenden Federstäben 15 angeordnet und wendelförmig um die beiden Federstäbe 15 des mittleren Schaftabschnitts 5 gewickelt und im weiteren Verlauf in Längsrichtung des proximalen Schaftabschnitts 6 abschnittweise, vorzugsweise punktuell, durch Schweißpunkte 18 miteinander verbunden und gegeneinander fixiert.

Zur Ausbildung der als Federstäbe 15 ausgebildeten Federelemente 14 können sowohl flache als auch runde Metallfedern, vorzugsweise aus Federstahl, verwendet werden. Runde Federstäbe 15 haben den Vorteil, dass sie beim Biegen keine Vorzugsrichtung haben, wohingegen flache Federstäbe 15 sich nur in zwei Richtungen leicht biegen lassen.

Bei den dargestellten Ausführungsbeispielen besteht der Endoskopschaft 2 aus drei Schaftabschnitten 4, 5 und 6. Alternativ ist es selbstverständlich auch möglich, den Schaft aus nur zwei Schaftabschnitten oder aus mehr als drei Schaftabschnitten aufzubauen. Vorteilhafterweise ist der Aufbau der einzelnen Schaftabschnitte so, dass der Flexibilitätsgrad der einzelnen Schaftabschnitte ausgehend vom flexibelsten distalen Schaftabschnitt 4 von distal nach proximal abnimmt, das heißt, dass die Schaftabschnitte immer unflexibler bzw. steifer werden.

Der Flexibilitätsgrad der einzelnen Schaftabschnitte 5 und 6 lässt sich durch die Materialwahl und Geometrie (Länge, Form, Durchmesser) der Federstäbe 15, die Anzahl der Federstäbe 15 und/oder die Anzahl der Wicklungen der Federstäbe 15 umeinander und/oder die Anzahl und Lage der Schweißpunkte 18 zueinander individuell einstellen.

Die wendelförmige Wicklung der Federstäbe 15 umeinander kann in jedem Schaftabschnitt 5, 6 mit gleicher oder unterschiedlicher Windungszahl und Windungssteigung ausgebildet sein. Mehr Windungen pro Längeneinheit bewirken dabei eine Versteifung des Schaftabschnittes 5 oder 6, wohingegen weniger Windungen pro Längeneinheit den jeweiligen Schaftabschnitt 5 oder 6 flexibler ausbilden.

Die Verwendung der Federstäbe 15 als Federelemente 14 ermöglicht eine einfache und kostengünstige Fertigung des flexiblen Endoskopschafts 2, so dass dieser sogar als Einwegartikel herstellbar und verwendbar ist.

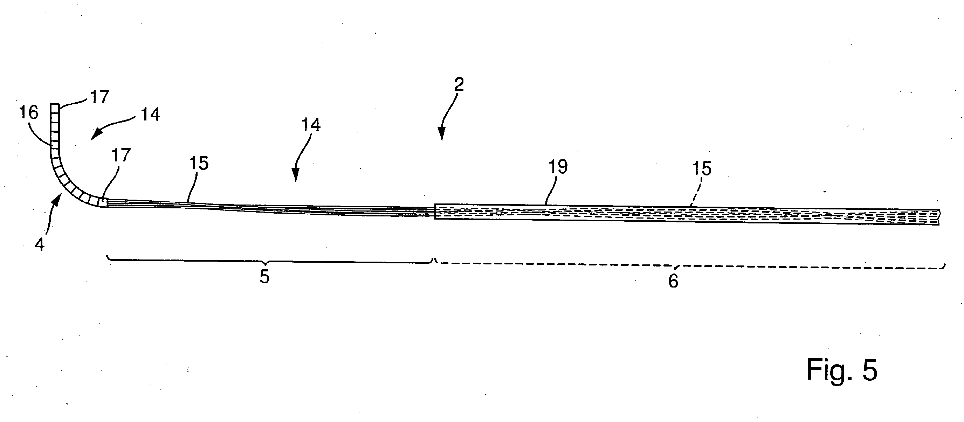

Wie in Abbildung Fig. 5 schematisch dargestellt, sind die Schaftabschnitte 4, 5 und 6 mit einem elastischen Außenmantel 19, vorzugsweise einem Schlauch, ummantelbar. Dieser Außenmantel 19 umschließt dann die Federelemente 14 und Segmente 16 sowie die koaxial zu den Federelementen 14 angeordneten Arbeitskanäle. Vorteilhafterweise ist der Außenmantel 19 so ausgebildet, dass die Arbeitskanäle in Längsrichtung des Endoskopschaftes 2 verlaufend direkt im Material des Außenmantels 19 ausgebildet sind, wodurch die Montage des Endoskopschafts 2 deutlich vereinfacht wird.

Gemäß einer alternativen Ausgestaltungsform des Endoskopschafts sind die Federelemente 14 und Segmente 16 sowie die koaxial zu den Federelementen 14 angeordneten Arbeitskanäle mit einer Art Netzstrumpf überzogen, der dann nachträglich von dem Außenmantel 19 umhüllt wird.

Ein wie voranstehend beschrieben aufgebauter flexibler Endoskopschaft 2 zeichnet sich dadurch aus, dass er bei einem konstruktiv einfachen und kostengünstigen Aufbau die erforderliche Flexibilität, insbesondere im distalen Schaftabschnitt 4, bei gleichzeitig ausreichender Biegefestigkeit, Schubstabilität und Torsionsstabilität aufweist.

**Bezugszeichenliste**

| | | | |
|---|---|---|---|
| 1 | Endoskop | 18 | Schweißpunkt |
| 2 | Endoskopschaft | 19 | Außenmantel |
| 3 | Handhabe | | |
| 4 | distaler Schaftabschnitt | | |
| 5 | mittlerer Schaftabschnitt | | |
| 6 | proximaler Schaftabschnitt | | |
| 7 | Zugmechanik | | |
| 8 | Steuereinrichtung | | |
| 9 | Stellhebel | | |
| 10 | Pfeil | | |
| 11 | Okular | | |
| 12 | Arbeitskanaleingang | | |
| 13 | Bowdenzug | | |
| 14 | Federelement | | |
| 15 | Federstab | | |
| 16 | Segment | | |
| 17 | Bowdenzugaufnahme | | |

## Patentansprüche

1. Flexibler Endoskopschaft mit einem Schaftkörper, der in Längsrichtung des Endoskopschaftes betrachtet aus mindestens zwei Schaftabschnitten (4, 5, 6) mit unterschiedlichem Flexibilitätsgrad besteht, wobei mindestens ein Schaftabschnitt (5, 6) aus mindestens einem Federelement (14) besteht und wobei alle Federelemente (14) der Schaftabschnitte (5, 6), die in Längsrichtung des Schaftkörpers betrachtet proximalseitig vom äußeren distalen Schaftabschnitt (4) angeordnet sind, als in Längsrichtung des Schaftkörpers verlaufende, reversibel verformbare Federstäbe (15) ausgebildet sind,
**dadurch gekennzeichnet,**
**dass** in Längsrichtung des Schaftkörpers betrachtet von distal nach proximal jeder der mindestens einen Federstab (15) aufweisenden Schaftabschnitte (5, 6) mindestens einen zusätzlichen Federstab (15) gegenüber dem mindestens einen Federstab (15) aufweisenden vorhergehenden Schaftabschnitt aufweist, der stoffschlüssig mit den anderen Federstäben (15) des jeweiligen Schaftabschnitts (5, 6) verbunden ist, wobei der mindestens eine zusätzliche Federstab (15) in Längsrichtung des Schaftkörpers verlaufend seitlich auf den anderen Federstäben (15) des jeweiligen Schaftabschnitts (5, 6) angeordnet ist.

2. Flexibler Endoskopschaft nach Anspruch 1, **dadurch gekennzeichnet, dass** jeder Schaftabschnitt (5, 6) der in Längsrichtung des Schaftkörpers betrachtet proximalseitig vom äußeren distalen Schaftabschnitt (4) angeordneten Schaftabschnitte (5, 6) mindestens zwei Federstäbe (15) aufweist, deren Längserstreckung jeweils bis zum proximalen Ende des Schaftkörpers reicht.

3. Flexibler Endoskopschaft nach Anspruch 1, **dadurch gekennzeichnet, dass** die Federstäbe (15) eines jeden Schaftabschnitts (5, 6) wendelförmig umeinander und/oder wendelförmig um die Federstäbe (15) der in Längsrichtung des Schaftkörpers betrachtet distal gelegenen Schaftabschnitte (4, 5) gewickelt sind.

4. Flexibler Endoskopschaft nach Anspruch 3, **dadurch gekennzeichnet, dass** die wendelförmig umeinander gewickelten Federstäbe (15) abschnittweise, vorzugsweise punktuell, stoffschlüssig miteinander verbunden sind.

5. Flexibler Endoskopschaft nach Anspruch 3 oder 4, **dadurch gekennzeichnet, dass** die Federstäbe (15) der einzelnen Schaftabschnitte (5, 6) mit gleicher oder unterschiedlicher Windungszahl um die Federstäbe (15) der in Längsrichtung des Schaftkörpers betrachtet distal oder proximal gelegenen Schaftabschnitte (4, 5) gewickelt sind.

6. Flexibler Endoskopschaft nach einem der Ansprüche 1 bis 5, **dadurch gekennzeichnet, dass** der Flexibilitätsgrad der einzelnen Schaftabschnitte (4, 5, 6) von distal nach proximal abnimmt.

7. Flexibler Endoskopschaft nach Anspruch 1 bis 6, **dadurch gekennzeichnet, dass** der äußerste distale Schaftabschnitt (4) aus einzelnen gelenkig miteinander verbundenen Segmenten (16) besteht.

8. Flexibler Endoskopschaft nach einem der Ansprüche 1 bis 7, **dadurch gekennzeichnet, dass** der äußerste distale Schaftabschnitt (4) über eine am distalen Schaftabschnitt (4) angreifende Zugmechanik (7) verschwenkbar ist.

9. Flexibler Endoskopschaft nach einem der Ansprüche 1 bis 8, **dadurch gekennzeichnet, dass** alle Schaftabschnitte (4, 5, 6) mit einem elastischen Außenmantel (19), vorzugsweise einem Schlauch, ummantelbar sind.

10. Flexibler Endoskopschaft nach Anspruch 9, **dadurch gekennzeichnet, dass** in dem Außenmantel (19) in Längsrichtung des Schaftkörpers verlaufende Arbeitskanäle angeordnet sind.

11. Endoskop, **gekennzeichnet durch** einen flexiblen Endoskopschaft (2) nach einem der Ansprüche 1 bis 10.

## Claims

1. A flexible endoscope shaft with a shaft body that, viewed in the longitudinal direction of the endoscope shaft, consists of at least two shaft sections (4, 5, 6) with different degrees of flexibility, wherein at least one shaft section (5, 6) consists of at least one spring element (14), and wherein all spring elements (14) of the shaft sections (5, 6), viewed in the longitudinal direction of the shaft body, are arranged on the proximal side from the outer, distal shaft section (4) and are constructed as reversible, deformable spring rods (15) running in the longitudinal direction of the shaft body,
**characterized in that**
viewed in the longitudinal direction of the shaft body from distal to proximal, each of the shaft sections (5, 6) comprising at least one spring rod (15) comprises at least one additional spring rod (15) opposite the previous shaft section comprising at least one spring rod (15), which shaft section is connected to and has the same substance as the other spring rods (15) of the particular shaft section (5, 6), wherein the at least one additional spring rod (15) is arranged running in the longitudinal direction of the shaft body and at the side of the other spring rods (15) of the particular shaft section (5, 6).

2. The flexible endoscope shaft according to Claim 1, **characterized in that** each shaft section (5, 6) of the shaft sections (5, 6) arranged, viewed in the longitudinal direction of the shaft body, on the proximal side of the outer, distal shaft section (4) comprises at least two spring rods (15) whose longitudinal extension extends to the proximal end of the shaft body.

3. The flexible endoscope shaft according to Claim 1, **characterized in that** the spring rods (15) of each shaft section (5, 6) are helically wound around each other and/or helically around the spring rods (15) of the distally located shaft sections (4, 5), viewed in the longitudinal direction of the shaft body.

4. The flexible endoscope shaft according to Claim 3, **characterized in that** the spring rods (15) that are helically wound around each other are preferably connected to each other in sections in a punctiform manner and consisting of the same substance.

5. The flexible endoscope shaft according to Claim 3 or 4, **characterized in that** the spring rods (15) of the individual shaft sections (5, 6) are wound with the same or different number of windings around the spring rods (15) of the shaft sections (4, 5) located distally or proximally viewed in the longitudinal direction of the shaft body.

6. The flexible endoscope shaft according to one of Claims 1 to 5, **characterized in that** the degree of flexibility of the individual shaft sections (4, 5, 6) decreases from distal to proximal.

7. The flexible endoscope shaft according to Claims 1 to 6, **characterized in that** the outermost distal shaft section (4) consists of individual segments (16) connected to each other in an articulated manner.

8. The flexible endoscope shaft according to one of Claims 1 to 7, **characterized in that** the outermost distal shaft section (4) can pivot via a traction mechanism (7) which attacks the distal shaft section (4).

9. The flexible endoscope shaft according to one of Claims 1 to 8, **characterized in that** all shaft sections (4, 5, 6) can be jacketed with an elastic outer jacket (19), preferably a hose.

10. The flexible endoscope shaft according to Claim 9, **characterized in that** work conduits running in the longitudinal direction of the shaft body are arranged in the outer jacket (19).

11. An endoscope, **characterized by** a flexible endoscope shaft (2) according to one of Claims 1 to 10.

## Revendications

1. Corps allongé flexible d'endoscope, comprenant un organe de corps allongé, qui, vu dans la direction longitudinale du corps allongé d'endoscope, est constitué d'au moins deux tronçons de corps allongé (4, 5, 6) avec des degrés de flexibilité différents, corps allongé d'endoscope
dans lequel au moins un tronçon de corps allongé (5, 6) est constitué d'au moins un élément de ressort (14),
et dans lequel tous les éléments de ressort (14) des tronçons de corps allongé (5, 6), qui, vu en direction longitudinale de l'organe de corps allongé, sont agencés du côté proximal du tronçon de corps allongé distal (4) extérieur, sont réalisés en tant que tiges de ressort (15) déformables de manière réversible et s'étendant dans la direction longitudinale de l'organe de corps allongé,
**caractérisé**
**en ce que**, vu dans la direction longitudinale de l'organe de corps allongé, en allant du côté distal vers le côté proximal, chacun des tronçons de corps allongé (5, 6) présentant au moins une tige de ressort (15), comporte par rapport au tronçon de corps allongé précédent présentant au moins une tige de ressort (15), au moins une tige de ressort (15) supplémentaire, qui est reliée par continuité de matière avec les autres tiges de ressort (15) du tronçon de corps allongé (5, 6) respectivement considéré, ladite au moins une tige de ressort (15) supplémentaire étant agencée, en s'étendant dans la direction longitudinale de l'organe de corps allongé, latéralement sur les autres tiges de ressort (15) du tronçon de corps allongé (5, 6) respectivement considéré.

2. Corps allongé flexible d'endoscope selon la revendication 1, **caractérisé en ce que** chaque tronçon de corps allongé (5, 6) des tronçons de corps allongé (5, 6) qui, vu dans la direction longitudinale de l'organe de corps allongé, sont agencés du côté proximal du tronçon de corps allongé distal (4) extérieur, comporte au moins deux tiges de ressort (15) dont l'étendue longitudinale respective va jusqu'à l'extrémité proximale de l'organe de corps allongé.

3. Corps allongé flexible d'endoscope selon la revendication 1, **caractérisé en ce que** les tiges de ressort (15) de chaque tronçon de corps allongé (5, 6) sont enroulées en forme d'hélice les unes autour des autres et/ou en forme d'hélice autour des tiges de ressort (15) des tronçons de corps allongé (4, 5) situés en position distale en se référant à la direction longitudinale de l'organe de corps allongé.

4. Corps allongé flexible d'endoscope selon la revendication 3, **caractérisé en ce que** les tiges de ressort (15) enroulées les unes autour des autres en forme d'hélice sont reliées mutuellement par tronçons, de préférence par points, par une liaison par continuité de matière.

5. Corps allongé flexible d'endoscope selon la revendication 3 ou la revendication 4, **caractérisé en ce que** les tiges de ressort (15) des tronçons de corps allongé (5, 6) individuels, sont enroulées, avec un nombre de spires identique ou différent, autour des tiges de ressort (15) des tronçons de corps allongé (4, 5) situés en position distale ou proximale en se référant à la direction longitudinale de l'organe de corps allongé.

6. Corps allongé flexible d'endoscope selon l'une des revendications 1 à 5, **caractérisé en ce que** le degré de flexibilité des tronçons de corps allongé (4, 5, 6) diminue en allant du côté distal vers le côté proximal.

7. Corps allongé flexible d'endoscope selon les revendications 1 à 6, **caractérisé en ce que** le tronçon de corps allongé distal (4) extérieur, est constitué de segments (16) individuels reliés les uns aux autres de manière articulée.

8. Corps allongé flexible d'endoscope selon l'une des revendications 1 à 7, **caractérisé en ce que** le tronçon de corps allongé distal (4) extérieur, peut pivoter par l'intermédiaire d'un mécanisme de traction (7) agissant sur le tronçon de corps allongé distal (4).

9. Corps allongé flexible d'endoscope selon l'une des revendications 1 à 8, **caractérisé en ce que** tous les tronçons de corps allongé (4, 5, 6) peuvent être enveloppés par une enveloppe extérieure élastique (19), de préférence sous la forme d'un tuyau flexible.

10. Corps allongé flexible d'endoscope selon la revendication 9, **caractérisé en ce que** dans l'enveloppe extérieure (19) sont agencés des canaux de travail s'étendant dans la direction longitudinale de l'organe de corps allongé.

11. Endoscope **caractérisé par** un corps allongé flexible d'endoscope (2) selon l'une des revendications 1 à 10.
